(19) Europäisches Patentamt
European Patent Office
Office européen des brevets

(11) Publication number: **0 270 023**
**A1**

(12) # EUROPEAN PATENT APPLICATION

(21) Application number: 87117564.2

(22) Date of filing: 27.11.87

(51) Int. Cl.⁴: **C07D 493/08**, C07D 311/02

(30) Priority: 03.12.86 US 937568

(43) Date of publication of application: 08.06.88 Bulletin 88/23

(84) Designated Contracting States: DE FR GB IT NL

(71) Applicant: Union Camp Corporation
1600 Valley Road
Wayne New Jersey 07470(US)

(72) Inventor: Mitchell, Peter W. D.
106 Lancaster Road
Freehold New Jersey(US)
Inventor: Sasser, David E.
6290 Nancy Drive
Jacksonville Florida(US)

(74) Representative: Boeters, Hans Dietrich, Dr. et al
Boeters, Bauer & Partner
Thomas-Wimmer-Ring 14
D-8000 München 22(DE)

(54) **Improved method of preparing cineoles.**

(57) A method is provided for preparing cineoles in an improved yield and with increased 1,4-cineole content, by reaction of terpin hydrate, terpineol or mixtures thereof with an acid, such as 50% sulfuric acid or 85% phosphoric acid, in the presence of a solvent for cineole which solvent is immiscible with the aqueous acid phase of the reaction mixture, and separating the cineole-containing solvent layer from the acid.

EP 0 270 023 A1

## IMPROVED METHOD OF PREPARING CINEOLES

The invention relates to a method for the preparation of cineoles by the rearrangement of a terpin hydrate or terpineol.

### BACKGROUND OF THE INVENTION

Cineoles are terpene ethers. Cineoles have been isolated from natural sources such as oils of wormwood and eucalyptus, or made synthetically from bicyclic terpene hydrocarbons, dipentenes, isoprenes, limonene, terpin hydrate, or terpineols. 1,4-Cineole (formula 1) is a by-product of the pine oil reaction

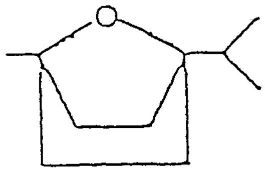
(1)

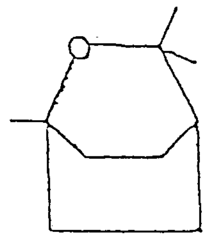
(2)

(the hydration of alpha-pinene under acid conditions). It occurs along with 1,8-cineole (formula 2) in about 12% of the crude product. The traditionally more desirable 1,8-cineole is also known as eucalyptol or 1,1,3-trimethyl-2-oxabicyclo[2.2.1]octane or 1,8-epoxy-p-menthane. The term cineole when used herein without numerical prefixes is inclusive of both the 1,4-and the 1,8-cineole isomers.

1,8-Cineole is a valuable aroma and pharmaceutical chemical, and much attention has been given to its synthesis, isolation, and purification. The technology of isolation of 1,8-cineole and one advantageous method for conducting this isolation is discussed in the patent of one of the present applicants, U. S. Patent 4,521,608 (1985). Much less is known about preparation and isolation of 1,4-cineole. The utility of 1,4-cineole as a synthetic intermediate has been recognized. For instance, it has recently been found to be a useful intermediate for the preparation of herbicides.

If good yields could be attained, it would be particularly attractive from a cost and availability standpoint to make cineoles from pine oil which is available domestically in large quantities from acid treatment of technical alpha-pinene. Applicants have found that 1,4-cineole is formed initially in greater amounts than 1,8-cineole in the formation of pine oil, but we have also found that 1,4-cineole is less stable than 1,8-cineole to the acid conditions of the pine oil forming reaction. It has been difficult to prepare cineole in good yield from pine oil by use of any known prior art methods, and it has been particularly difficult to prepare 1,4-cineole from that source. Fractional distillation to separate 1,4-from 1,8-cineole has been found quite difficult, if not commercially infeasible, unless means can be found for supplying a feed to the still having the 1,4-isomer substantially enriched relative to the 1,8 - isomer.

The use of solvents to separate cineoles from each other or from by-products is also known in the art. U.S. Patent 2,315,986 describes the use of phenol and U.S. Patent 3,923,837 describes the use of concentrated sulfuric acid as solvents to be added to the reaction mixture after the conversion reaction is completed to separate the reaction products.

The rearrangement of terpineols in the presence of mineral acid is a known procedure for making cineoles; see for example U.S. Patent 1,994,131. It was shown to be necessary to control carefully the temperature, pressure, and acid concentration to avoid the undesired dehydration of the terpineol to an olefin. However, even with the selection of optimum rearrangement conditions, the yields of cineole from terpineols were quite low.

It would be highly desirable if an improvement in the prior art method of preparing cineole could be provided which would result in greater overall yields of cineole and particularly an improvement which would make possible the synthesis of the less-acid-stable isomer of cineole, namely 1,4-cineole, in much better yield than hitherto possible.

## SUMMARY OF THE INVENTION

An improved method is disclosed for preparing cineoles which compromises rearranging a terpineol or partially dehydrating and rearranging terpin hydrate, by carrying out the rearrangement of terpineol or partial dehydration and rearrangement of terpin hydrate, in the presence of a solvent for the cineole which is immiscible with the aqueous acid phase of the rearrangement reaction mixture. The method of the invention provides improved yields of cineole product, and in particular, much improved yields of the less-acid-stable isomer, 1,4-cineole.

## DETAILED DESCRIPTION OF THE INVENTION

The method of the invention is carried out by the reaction of a terpene alcohol selected from the group consisting of terpin hydrate and a terpineol and mixtures thereof with a strong acid in the presence of a solvent for the cineole product; said solvent being substantially immiscible with the aqueous acid phase of the reaction mixture. The use of the word "reaction" or "reacting" in the present case is meant to include the participation of the acid in a catalytic mode, wherein it is not consumed stoichiometrically.

Terpin hydrate as used herein refers to a saturated terpene diol which is produced, for example, from oil of turpentine. It is the hydrate of cis-terpin, or cis-p-menthane-1,8-diol. Obviously, the anhydrous form could be used as well, but in the water-containing acid reaction medium it would presumably be hydrated.

Terpineol as used hereinafter refers to any of the monounsaturated terpene monohydric alcohols, including but not limited to alpha-terpineol, beta-terpineol, delta-terpineol, gamma-terpineol, terpinen-1-ol, terpinen-4-ol, p-menth-2-en-8-ol, p-menth-8-en-1-ol, p-menth-3-en-1-ol, p-menth-1(7)-en-4-ol, or mixtures thereof such as occur in "produced pine oil," or "pine oil and heavier," technical products made by hydration of pinene using acid catalyst. It will be evident to one skilled in the art of terpene chemistry that acid catalyzed reactions of terpene alcohols are replete with double bond and hydroxy group rearrangements and many isomers of the starting terpineol can yield the same final product. It will also be evident that the process of the invention could be run subsequently to the pinene hydration in the same vessel, and using either a fresh charge of acid or the acid left from the pinene hydration step boosted if necessary to the higher concentration preferred for the cineole-forming step.

It is believed that under the conditions of the reaction to form cineole, terpin hydrate undergoes dehydration to form terpineols, so that the cineole-forming process starting from terpin hydrate may proceed mainly or to a large degree by way of terpineols as intermediates, since it is known that terpin hydrate can react with acids to form terpineols (see for example, Bedoukian, "Perfume and Flavoring Synthesis," 2nd Edition, 1967, p. 340). It should be noted that the present invention is not limited to the stated hypothesis, but only by the claims appended hereto especially since the relative rates of the dehydration and the rearrangement steps are not known with any degree of certainty.

The relative advantages of the use of terpin hydrate compared to the use of terpineols as starting materials will depend on cost and engineering considerations. Terpin hydrate is somewhat easier to isolate in good yield from pine oil. On the other hand, since the best cineole yields are obtained at partial conversion where terpineols will be recovered for recycle, it would be an extra step to take the terpineols back to terpin hydrate even if this were feasible, or alternatively, a complex flow sequence would be required with terpin hydrate being fed at one point and recycle terpineols being fed at another point. Moreover, since starting with terpineols results in no water formation in the cineole-forming reaction, the aqueous acid is subjected to less dilution. Thus, starting with terpineols permits use of lower acid strengths to begin with, and simplifies the recovery and recycle of the acid. Obviously, since either terpin hydrate or terpineol can be used in the process of the invention, a mixture of them can be used also.

It has unexpectedly been found that by selection of certain terpineols or a feed rich in certain terpineols, the level of 1,4-cineole relative to 1,8-cineole can be raised, thus greatly simplifying the purification of the 1,4-cineole. In particular, by selection of terpinen-1-ol, terpinen-4-ol, or to a lesser extent beta-terpineol the yield of 1,4-cineole can be much higher than the yield of 1,8-cineole, up to 10 times as high. By contrast, using a typical alpha-terpineol as starting material, the ratio of 1,4-to 1,8-cineole may be in the vicinity of 1.9:1.

The strong acid to be used in the process of the invention can be a mineral acid, such as sulfuric, phosphoric, sulfamic, or a strong organic acid such as toluenesulfonic, xylenesulfonic, methylsulfonic, oxalic, formic, chloroacetic, or methyl phosphoric, to cite a few examples. The acid should not be one which is miscible in the organic solvent and in order to attain immiscibility, some water may have to be added. The preferred acids are sulfuric acid in the range of 30-75% and phosphoric acid in the range of 50-90%,

and the mole ratio of acid to terpene alcohol being in the range of 10:1 to 1:10. Where terpin hydrate is used as the terpene alcohol reactant, about 2 moles acid to 1 mole of terpin hydrate is preferred. Sulfuric acid in the vicinity of 50% concentration is most preferred because of maximum yields obtained with its use when the reaction temperature is in the vicinity of ambient (10-35°C).

The organic solvent used in the process of the invention should be one which is miscible with cineoles, but not with the acid phase of the reaction mixture, by which is meant a phase comprising the acid, any water added or formed, any dissolved terpene alcohols and any protonated reaction intermediates. The solvent is believed to capture the cineole product and withdraw it from the reaction zone to save it from yield-reducing further reactions such as dehydration; however, patentability is not to be limited to this theory. Empirically, the presence of the solvent increases the yield substantially.

Suitable solvents include but are not limited to aliphatic hydrocarbons such as hexane, octane, kerosine, petroleum naphtha, high flash naphtha, or eicosane, aromatic hydrocarbons such as benzene, toluene, xylene, cumene, methylnaphthalene, heavy aromatic naphtha, and the like, terpene hydrocarbons, halogenated hydrocarbons such as methylene chloride, chloroform, carbon tetrachloride, dichloropropane, chlorobenzenes, ethers such as diethyl ether, diisopropyl ether, anisole, diphenyl ether, bis(2-chloroethyl) ether, esters such as ethyl acetate, butyl acetate, methyl benzoate, dibutyl phthalate, dioctyl phthalate, or diethyl carbonate. Preferred solvents are toluene and xylene because of good yields, economy, and environmental advantages, and because their boiling points are sufficiently removed from that of cineole so as to facilitate separation by distillation. It is preferred that the solvent have a boiling point at least 10°C away from the boiling point of cineole so as to facilitate separation by distillation. The quantity of solvent is not critical, it being only necessary that it should produce a separate phase from the rest of the reaction mixture, in other words, it should be substantially immiscible with the acid used in the reaction, and it should dissolve the cineole produced in the reaction. The solvent weight can be from about 10% to 1000% of the weight of the terpene alcohol charged, preferably about 20% to 500% of the weight of the terpene alcohol charged.

The reaction temperature can be from -20°C to 100°C, preferably from 0 to 80°C. Temperatures at or near ambient (10-35°C) can conveniently be used, thus simplifying the process equipment requirements. Reaction times range from a few seconds at the higher end of the temperature range to several days at the low end of the temperature range, most typically 10 minutes to 24 hours.

The reaction can be carried out batchwise or continuous. If continuous, it can be done in a stirred vessel with continuous feed of the terpene alcohol, the solvent, or mixture thereof, and the acid, and with continuous overflow of products. A cascade of two or more such vessels can be used to avoid by-passing of feed in continuous mode. The reaction can also be carried out in a column or pipe with mixing means such as a circulating pumps, axial stirrers, or in-line static mixers.

The product at the end of the reaction is recovered by separating the organic solvent layer in which the product is dissolved, optionally washing it with water or aqueous base to remove acid, and distilling to separate the solvent (for possible recycle), the cineole product, and any unreacted terpene alcohols for possible recycle.

Various schemes, including those in the prior art, can be used to separate 1,4-and 1,8-cineole. These include fractional distillation and selective complexation with hydroquinone or with zeolites. Various methods are reviewed by Goldstein in U.S. Patent 4,347,189 (1982), col. 2.

The following examples describe the manner and process of the invention and set forth the best mode contemplated by the inventors for carrying out the invention, but are not to be construed as limiting.

EXAMPLE 1

A mixture of 100 g of pine oil (primarily a mixture of terpineols), 225 g of toluene, and 285 g of 50% aqueous sulfuric acid was stirred at room temperature and periodically analyzed by gas chromatography (hereinafter abbreviated gc). After 1½ hours, the mixture contained 8.2% 1,4-cineole and 4.1% 1,8-cineole, and after 4½ hours 7.0% 1,4-cineole and 4.4% 1,8-cineole, in each case 70% toluene. Based on area % gc analysis, this is approximately a 36-39% weight yield of cineoles:

EXAMPLE 2

A mixture of 100 g mixed terpineol, 225 g toluene, and 356 g 40% aqueous sulfuric acid was stirred at 25-30 deg. C and sampled from time to time to determine the point at which 1,4-cineole was maximized. This was found to be after about 14 hours at which point gc analysis showed 9.7% 1,4-cineole and 4.7% 1,8-cineole. At 16 hours, the assay had declined just slightly to 9.4 and 4.7% respectively. The layers were separated, the toluene layer was washed with 5% NaOH and water, and dried over potassium carbonate. It was reanalyzed and found to have 9.1% 1,4-cineole and 4.7% 1,8-cineole, and 73% toluene, corresponding to a 42% weight yield of cineoles based on terpineol charged.

In a further experiment similar to the above, using 40% sulfuric acid, it was found that a ratio of 1,4-cineole to total dipentenes of 52% was obtained at the point where about 65% of the terpineol was converted, whereas the ratio of 1,4-cineole to total dipentenes dropped to 45.5% when about 80% of the terpineol was converted. This result shows,that for a maximum selectivity, the process should be run to an intermediate conversion with recycle of unreacted terpineol.

EXAMPLE 3

A mixture of 100 g of terpineol, 225 g of toluene, and 169 g of 75% phosphoric acid was stirred at 25-30 deg. C and sampled periodically to determine maximum cineole, which occurred in about 10 hours. The toluene layer was worked up as in the preceding example and found to contain 7.3% 1,4-cineole and 5.5% 1,8-cineole and 72.7% toluene corresponding to a 40% weight yield of cineoles.

In a further experiment similar to the above, with phosphoric acid catalyst, the maximum ratio of 1,4-cineole to dipentenes is obtained at a partial conversion of terpineol of approximately 43%.

A technical crude terpineol fraction called "pine oil and heavier" was found to give similar results to those described above in this example.

EXAMPLE 4

A mixture of 50 g of terpin hydrate, 107.5 g. of 53% sulfuric acid and 91 g of toluene was stirred at 28-33 deg. C for 22.5 hours. A second similar mixture was stirred at 24-25 for 3.5 hours. The first run showed 33% yield of 1,4-cineole and the second run showed a 37.6% yield of 1,4-cineole, both superior to yields from prior art methods.

EXAMPLE 5

A series of runs was conducted by stirring 10 g of terpin hydrate, 18.2 g of toluene, and 11.2 g of sulfuric acid plus the indicated amount of water at room temperature for 17 hours, to determine effect of acid concentration; results as follows:

| | | | | |
|---|---|---|---|---|
| Water (g): | 0 | 5 | 10 | 15 |
| Acid concentration (wt. %) | 98% | 68% | 52% | 42% |
| Mole % yield 1,4-cineole: | 0% | 0% | 34.4% | 10.2% |

A series of repeat runs using 10 g. water gave 38.2-42.1 mole % yields of 1,4-cineole, showing consistently improved yields.

EXAMPLE 6

A series of runs was made with terpin hydrate, phosphoric acid, and toluene to ascertain the effect of the amount and concentration of phosphoric acid and the effect of reaction time. The standard conditions involved stirring 12.9 g (68 mmoles) of terpin hydrate, 16.3 g of phosphoric acid monohydrate (140 mmoles), and 23.5 g of toluene for 19 hours at room temperature. The toluene solution was found by gc analysis to have 10.07 wt % 1,4-cineole corresponding to a 34.5 mole % yield. Then a series of similar runs was made varying the mole ratio of acid to terpin hydrate. The results were as follows:

| | | | | |
|---|---|---|---|---|
| Mole ratio: | 3.9 | 2.2 | 1.1 | 0.6 |
| Mole % yield | 3.7% | 29.3% | 11.4% | 7.7% |

This experiment showed a critical amount of acid for best results.

A series of similar experiments was conducted at a fixed mole ratio of 4.0 mole acid per mole of terpin hydrate. Samples were taken at various time intervals, with the following results:

| | | | | | | |
|---|---|---|---|---|---|---|
| Time (hours): | 0.5 | 1 | 2 | 3 | 4 | 5 |
| Wt. % 1,4-cineole: | 2.3% | 4.8% | 8.2% | 6.8% | 5.7% | 4.2% |

The 8.2 wt. % 1,4-cineole result corresponds to 23.8 mole % yield. These data show that selecting an optimum reaction time maximizes yield.

A series of similar experiments was run to examine the effect of acid concentration. A constant mole ratio of 2.2 moles acid per mole of terpin hydrate was used. The reactions were run for 17 hours at room temperature.

| | | | | |
|---|---|---|---|---|
| Conc. (%): | 85 | 62 | 48 | 40 |
| Mole % yield: | 26.9 | 2.4 | 0 | 0 |

This data shows that with phosphoric acid, a concentration of at least about 60% is needed under these reaction conditions. At higher reaction temperatures the more dilute phosphoric acid can be used. Spent acid having too low a phosphoric acid concentration to be effective under the given reaction conditions can be concentrated by evaporation. The dilute phosphoric acid can also be used in the pine oil synthesis reaction.

EXAMPLE 7

A mixture of 50 g of terpin hydrate, 91 g of toluene, and 75 g of a mixed toluenesulfonic/xylenesulfonic acid was stirred for 18 hours at 25-35 deg. C. The toluene layer was separated and found to contain 14.3% 1,4-cineole, 9.2% 1,8-cineole, 68% dipentenes on a toluene-free basis.

EXAMPLE 8

Terpin hydrate (400 g, 2.105 moles), 714 g of toluene, and 848 g (4.48 moles) of 52% sulfuric acid were stirred for 20 hours at ambient temperatures (25°C).

Separation of the phases gave 940.9 g of acid analyzing 45 wt. % sulfuric acid and 1016.6 g of toluene solution analyzing 12.8 wt. % 1,4-cineole and 6.9 wt. % 1,8-cineole. This represents a 40.1 mole % yield of 1,4-cineole and a 61.7 mole % yield of total cineoles.

Toluene was removed at atmospheric pressure leaving 449 g of oil which was fractionated to give 259 g of cineole-containing fractions which by analysis contained 105.8 g of 1,4-cineole or a yield of 32.7 mole %. Analysis of the toluene removed showed that the remaining 1,4-cineole had codistilled with the toluene.

EXAMPLE 9

A mixture of 100 parts by weight of a technical terpineol fraction called in the terpene trade "pine oil and heavier," 210 parts of solvent as specified in the table below, and 180 parts by weight of 65% phosphoric acid was stirred at 40-42°C and sampled from time to time to determine the point at which the 1,4-cineole was maximized. At that point, the layers were separated, the solvent layer was washed with 5% sodium hydroxide solution and with water, and dried over anhydrous potassium carbonate. The table below lists the results obtained for a number of solvents:

| Solvent | Reaction Time (hrs) | Conversion % | 1,4-cineole Selectivity | 1,8-cineole Selectivity |
|---|---|---|---|---|
| Toluene | 12 | 55.7 | 44.5 | 20.4 |
| Xylene | 5 | 57.6 | 44.6 | 20.9 |
| n-Tetradecane | 6 | 76.3 | 38.7 | 19.1 |
| Caryophyllene | 9 | 69.5 | 39.2 | 19.8 |
| Aromatic 200 | 7.5 | 50.0 | 44.8 | 20.0 |
| Norpar 15 | 9 | 81.3 | 38.9 | 18.7 |
| Isopar V | 6 | 47.1 | 40.8 | 18.9 |
| Exxon D110 | 4.5 | 72.1 | 37.1 | 19.1 |
| Alpha-olefin | 4.5 | 83.3 | 38.8 | 18.9 |
| Decanol | 8 | 33.0 | 34.2 | 20.2 |

Notes on above: Aromatic 200 is a heavy aromatic petroleum solvent available from Exxon Corp. Norpar 15 is a treated normal paraffin solvent available from Exxon Corp. Isopar V is a petroleum solvent consisting of C14-C18 isoparaffinic hydrocarbons available from Exxon Corp. Exxon D110 is a hydrotreated light petroleum distillate from Exxon. The alpha-olefin is a mixture of 1-tetradecene and 1-hexadecene available from Ethyl Corp. The selectivity figures given are percentages relative to all hydrocarbons and cineole generated.

EXAMPLE 10

A mixture of 500 g of terpinen-1-ol (65% this isomer, 88% total terpineols), 1040 g toluene, and 1480 g of 65% phosphoric acid was stirred at 40-42°C and sampled from time to time to determine the point at which 1,4-cineole was maximized. At this point, the layers were separated, the toluene layer was washed with 5% sodium hydroxide and water, and dried over potassium carbonate. The 1,4-cineole content was found to maximize at 50.5% at a conversion of 48.7%. The selectivity of 1,8-cineole at this conversion was 3.6%.

Under these same conditions, starting with alpha-terpineol (82% assay, 96.5% total terpineols), maximum selectivities of 1,4-cineole of 44.5% and 1,8-cineole of 23.7% were obtained at a conversion of 69.1%.

**Claims**

1. In the method of preparing cineole wherein a terpene alcohol selected from the group consisting of terpin hydrate terpineol and mixtures thereof, is reacted with a strong acid, the improvement which comprises: carrying out the reaction in the presence of a solvent for the cineole which is substantially immiscible with the reaction mixture of the terpene alcohol and the strong acid.

2. The method of claim 1 where the acid is sulfuric acid or phosphoric acid.

3. The method of claim 1 or 2 where the terpene alcohol is terpin hydrate or a terpineol.

4. The method of any of the preceding claims where the solvent is an aromatic hydrocarbon preferably toluene.

5. A method of preparing cineole in which at least one terpene alcohol selected from the group consisting of terpin hydrate and a terpineol is reacted with approximately 50 % sulfuric acid in the presence of a solvent for cineole which is immiscible with the aqueous acid phase at a temperature near ambient until the cineole concentration has substantially reached a maximum, and separating the cineole containing solvent layer from the acid.

6. A process for the preparation of cineole having an enhanced content of 1,4-cineole which comprises reacting at least one terpene alcohol selected from the group consisting of terpin hydrate and terpineol with sulfuric acid of about 50 % concentration at substantially ambient temperature in the presence of a solvent for cineole said solvent being substantially immiscible with the acid component of the reaction mixture, and separating said solvent and cineole from the reaction mixture when the 1,4-cineole content has substantially reached a maximum, and isolating the cineole from said solvent.

7. A process for the preparation of cineole having an enhanced content of 1.4-cineole which comprises reacting at least one terpene alcohol selected from the group consisting of terpinen-1-o1, terpinen-4-o1, and beta-terpineol with a strong acid in the presence of a solvent for cineole, said solvent being substantially immiscible with the acid component of the reaction mixture.

8. The process of claim 7 wherein the terpene alcohol is terpinen-1-o1 or terpinen-4-o1 or beta-terpineol.

9. The process of claim 7 or 8 wherein the strong acid is phosphoric acid.

10. A process For the preparation of cineole having an enhanded content of 1,4-cineole which comprises reacting at least one terpene alcohol selected from the group consisting of terpinen-1-o1, terpinen-4-o1, and beta-terpineol, with phosphoric acid of about 65 % concentration at substantially ambient temperature in the presence of a solvent for cineole said solvent being substantially immiscible with the acid component of the reaction mixture, and separating said solvent and cineole from the reaction mixture when the 1,4-cineole content has substantially reached a maximum, and isolating the cineole from said solvent.

European Patent Office

# EUROPEAN SEARCH REPORT

Application number

EP 87117564.2

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,A | EP - A2/A3 - 0 125 539 (UNION CAMP CORPORATION)<br>* Abstract *<br>-- | 1 | C 07 D 493/08<br>C 07 D 311/02 |
| A | US - A - 4 347 189 (GOLDSTEIN)<br>* Abstract *<br>-- | 1 | |
| A | CHEMICAL ABSTRACTS, vol. 91, no. 7, August 13, 1979, Columbus, Ohio, USA<br>TAKAHASHI, H.; ITO, M. "Synthesis of 7-oxabicyclo[2.2.1]heptanes from 3-cyclohexenols involving photo-induced hypoiodite reaction" page 727, column 2, abstract-no. 57 190v<br>& Chem. Lett. 1979, (4), 373-4<br>-- | 1 | TECHNICAL FIELDS SEARCHED (Int. Cl.4) |
| A | CHEMICAL ABSTRACTS, vol. 70, no. 13, March 31, 1969, Columbus, Ohio, USA<br>AIKAWA, T.; SHIIHARA, Y.; SANO, H.; IZUMITANI, H. "Cineules" page 400, column 2, abstract-no. 58 058m<br>& JP-68-24 186<br>-- | 1 | C 07 D 493/00<br>C 07 D 311/00 |

The present search report has been drawn up for all claims

| Place of search | Date of completion of the search | Examiner |
|---|---|---|
| VIENNA | 09-02-1988 | BRUS |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application number



EP 87117564.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | CHEMICAL ABSTRACTS, vol. 82, no. 9, March 3, 1975, Columbus, Ohio, USA<br>BARDYSHEV, I.I.; SEDEL'NIKOV, A.I.; TIKHONOVA, T.S. "Reactions of dipentene under the action of aqueous solutions of acids" page 594, column 1, abstract-no. 57 954m<br>& Vestsi Akad. Navuk B. SSR, Ser. Khim. Navuk 1974, (5), 61-4<br>-- | 1 | |
| A | CHEMICAL ABSTRACTS, vol. 82, no. 17, April 28, 1975, Columbus, Ohio, USA<br>BARDYSHEV, I.I.; SEDEL'NIKOV, A.I.; TIKHONOVA, T.S. "Chemical transformations of dipentene caused by aqueous solutions of sulfuric acid. II. Reaction Kinetics" page 523, column 1, abstract-no. 112 167g<br>& Vestsi Akad. Navuk B. SSR, Ser. Khim. Navuk 1974, (6), 17-19<br>---- | 1 | TECHNICAL FIELDS SEARCHED (Int. Cl.4) |

The present search report has been drawn up for all claims

| Place of search | Date of completion of the search | Examiner |
|---|---|---|
| VIENNA | 09-02-1988 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document